# EUROPÄISCHE PATENTANMELDUNG

(11) **EP 2 881 665 A1**
(43) Veröffentlichungstag der Anmeldung: **10.06.2015**
(21) Anmeldenummer: 14178289.6
(22) Anmeldetag: 24.07.2014
(51) Int. Cl.: F23N 5/12, G01N 25/22, G01N 33/22

(54) **Verfahren und Vorrichtung zur Bestimmung von Kennwerten von Brenngasen**

(30) Priorität: 26.07.2013 DE 102013214610
(71) Anmelder: E.ON Technologies GmbH, 45896 Gelsenkirchen (DE)
(72) Erfinder: Nitschke-Kowsky, Dr. Petra, 46284 Dorsten (DE); Dahlhaus, Dipl.-Ing. Johannes, 48249 Dülmen (DE)
(74) Vertreter: Glawe, Delfs, Moll

(57) **Zusammenfassung**

Ein Verfahren und eine Vorrichtung zur Bestimmung wenigstens eines Kennwerts von Brenngasen. Das erfindungsgemäße Verfahren umfasst die Schritte:
a. Vermischen eines kontinuierlichen Volumenstroms von Luft und eines kontinuierlichen Volumenstroms eines Brenngases, wobei ein oder beide Volumenströme über einen Messzeitraum derart kontinuierlich verändert werden, dass zu einem ersten Zeitpunkt im Messzeitraum ein unterstöchiometrisches Gemisch und zu einem zweiten Zeitpunkt im Messzeitraum ein überstöchiometrisches Gemisch vorliegt;
b. Verbrennen des Gemisches bei gleichzeitiger Überwachung der Flamme durch einen Sensor (40); und
c. Ermitteln des Luftbedarfs des Brenngases aus dem Verhältnis der Volumenströme von Luft und Brenngas zum Zeitpunkt (t₂) einer über den Sensor (40) festgestellten stöchiometrischen Verbrennung.

Die erfindungsgemäße Vorrichtung ist zur Durchführung des erfindungsgemäßen Verfahrens ausgebildet.

## Beschreibung

Die Erfindung betrifft ein Verfahren und eine Vorrichtung zur Bestimmung wenigstens eines Kennwerts von Brenngasen.

Unter anderem in Industriebetrieben, aber auch im Privatbereich wird Brenngas verwendet, um definiert an vorgesehenen Punkten oder in bestimmten Vorrichtungen gezielt Wärme zu erzeugen, bspw. an Dampf- oder Heizkesseln. Hierzu werden häufig einstellbare Gasbrenner verwendet, bei denen sich die Gas- und/oder Luftmenge für die Verbrennung einstellen lassen.

Für eine optimale Verbrennung hinsichtlich Flammenstabilität, Emissionen und Effizienz ist es erforderlich das Verhältnis von Gas zu Luft genau einzustellen. Bei wechselnden Gasbeschaffenheiten kann die einzustellende Luftzahl dabei von den Kennwerten des zum Einstellzeitpunkt fließenden Gases abhängen.

Um das optimale Gas-Luft-Verhältnis für die Verbrennung eines Gases bspw. in einem Gasbrenner einstellen zu können, müssen vom Gas Kennwerte bekannt sein, die das Gas hierfür hinreichend charakterisieren. Bei einem hierfür geeigneten Kennwert kann es sich bspw. um den Luftbedarf für die Verbrennung des Gases handeln. Entsprechende Kennwerte sind häufig nicht unmittelbar bekannt.

Der Erfindung liegt die Aufgabe zugrunde, ein Verfahren und eine Vorrichtung zu schaffen, mit der sich wenigstens ein Kennwert von Brenngasen bestimmen lässt.

Diese Aufgabe wird gelöst durch ein Verfahren gemäß dem Hauptanspruch sowie eine Vorrichtung gemäß dem unabhängigen Anspruch 8. Vorteilhafte Weiterbildungen sind Gegenstand der abhängigen Ansprüche.

Demnach betrifft die Erfindung ein Verfahren zur Bestimmung wenigstens eines Kennwerts von Brenngasen umfassend die Schritte:
a. Vermischen eines kontinuierlichen Volumenstroms von Luft und eines kontinuierlichen Volumenstroms eines Brenngases, wobei ein oder beide Volumenströme über einen Messzeitraum derart kontinuierlich verändert werden, dass zu einem ersten Zeitpunkt im Messzeitraum ein unterstöchiometrisches Gemisch und zu einem zweiten Zeitpunkt im Messzeitraum ein überstöchiometrisches Gemisch vorliegt;
b. Verbrennen des Gemisches bei gleichzeitiger Überwachung der Flamme durch einen Sensor; und
c. Ermitteln des Luftbedarfs des Brenngases aus dem Verhältnis der Volumenströme von Luft und Brenngas zum Zeitpunkt einer über den Sensor festgestellten stöchiometrischen Verbrennung.

Die Erfindung betrifft weiterhin eine Vorrichtung zur Bestimmung wenigstens eines Kennwerts von Brenngasen umfassend zwei voneinander getrennte, veränderbare Messvolumen für Luft und Brenngas, die mit einem Brenner verbunden sind, ein im Flammenbereich des Brenners angeordneter Sensor und eine Steuervorrichtung, die mit dem Sensor verbunden ist, wobei die Steuervorrichtung zum Steuern der Volumenänderung der Messvolumen in einem Messzeitraum ausgebildet ist, sodass Luft und Brenngas jeweils als kontinuierlicher Volumenstrom den Brenner zugeführt und ein oder beide Volumenströme im Messzeitraum derart kontinuierlich verändert werden, dass zu einem Zeitpunkt im Messzeitraum ein unterstöchiometrisches Gemisch und zu einem anderen Zeitpunkt im Messzeitraum ein überstöchiometrisches Gemisch vorliegt und der Luftbedarf des Brenngases aus dem Verhältnis der Volumenströme von Luft und Brenngas zum Zeitpunkt einer über den Sensor festgestellten stöchiometrischen Verbrennung ermittelt wird.

Zunächst werden einige im Rahmen der Erfindung verwendete Begriffe erläutert:
Als "kontinuierlicher Volumenstrom" wird ein grundsätzlich unterbrechungsfreier Volumenstrom verstanden. Stoß- oder impulsartige Volumenströme, bei denen sich Phasen des Bewegens und des Stillstehens eines Volumens des strömenden Mediums vorhanden sind oder abwechseln, gelten nicht als kontinuierlicher Volumenstrom.

Eine "kontinuierliche Veränderung eines Volumenstroms" bedeutet eine nicht-sprunghafte Änderung des Volumenstroms, sodass jeder zwischen einem Startwert und einem Zielwert liegende Wert bei der Veränderung des Volumenstroms durchlaufen wird. Es ist möglich, innerhalb einer kontinuierlichen Veränderung eines Volumenstroms Phasen mit einem konstanten Volumenstrom vorzusehen.

Eine stöchiometrische Verbrennung liegt vor, wenn bei einer vollkommenen Verbrennung des einem Brenner zugeführten Brenngases der Sauerstoff aus der ebenfalls zugeführten Luft vollständig aufgebraucht wird, es für die Verbrennung also weder an Sauerstoff fehlt noch dass Sauerstoff nach der Verbrennung übrig bleibt. Bei einem "unterstöchiometrischen Gemisch" aus Brenngas und Luft ist der Brenngasanteil am Gemisch höher als bei einem stöchiometrischen Gemisch, sodass das Brenngas aufgrund von Sauerstoffmangel nicht vollständig verbrennt. Bei einem "überstöchiometrischen Gemisch" ist anteilig mehr Luft als notwendig vorhanden, sodass selbst bei einer vollständigen Verbrennung des Brenngases noch Sauerstoff übrig bleibt.

Die vorliegende Erfindung ermöglicht es, ohne nähere Kenntnis von Art und Beschaffenheit eines Brenngases für dessen Verbrennung wenigstens einen Kennwert, nämlich den Luftbedarf, einfach zu ermitteln. Dazu wird über die Volumenströme von Brenngas und Luft, von denen zumindest einer veränderbar ist, das Brenngas/Luft-Verhältnis des Gemisches für die Verbrennung kontrolliert variiert. Das Verhältnis wird dabei derart verändert, dass zu einem Zeitpunkt im Messzeitraum ein unterstöchiometrisches, zu einem anderen Zeitpunkt ein überstöchiometrisches Gemisch für die Verbrennung vorliegt. Das Verhältnis kann dabei durch Veränderung beider Volumenströme verändert werden. Es ist aber auch möglich, das Verhältnis durch die Veränderung eines einzelnen Volumenstroms zu bewerkstelligen; der andere Volumenstrom ist in diesem Fall vorzugsweise konstant.

Aufgrund der kontinuierlichen Veränderung des einen oder beider Volumenströme ist weiterhin sichergestellt, dass zu einem Zeitpunkt im Messzeitraum ein stöchiometrisches Verhältnis von Brenngas und Luft vorliegt. Sofern nämlich durch eine kontinuierliche Veränderung des oder der Volumenströme und damit dem Brenngas/Luft-Verhältnis des Gemisches ein unterstöchiometrisches Verhältnis zu einem überstöchiometrischen Verhältnis (oder umgekehrt) überführt wird, liegt zwangsläufig wenigstens kurzzeitig ein stöchiometrisches Verhältnis von Brenngas und Luft vor. Dabei ist grundsätzlich unerheblich, ob im Messzeitraum zunächst ein überstöchiometrisches oder ein unterstöchiometrisches Gemisch vorliegt und die kontinuierliche Veränderung der Volumenströme dann zu einem unter- bzw. überstöchiometrischen Gemisch führt. In beiden Fällen liegt wenigstens kurzzeitig ein stöchiometrisches Verhältnis vor.

Das Vorliegen des stöchiometrischen Verhältnisses während der kontinuierlichen Veränderung des oder der Volumenströme kann über den Sensor, der die Flamme überwacht, festgestellt werden. Aus den Volumenströmen von Luft und Brenngas zum Zeitpunkt der durch den Sensor festgestellten stöchiometrischen Verbrennung lässt sich der Luftbedarf für eine stöchiometrische Verbrennung des Brenngases ermitteln.

Der so für ein bestimmtes Brenngas ermittelte Luftbedarf für eine stöchiometrische Verbrennung kann dann als Grundlage verwendet werden, um mit diesem Brenngas betriebene Vorrichtungen, beispielsweise Gasbrenner, zumindest hinsichtlich des Brenngas/Luft-Verhältnisses einzustellen.

Es ist bevorzugt, wenn der Volumenstrom des Brenngases oder der Luft über den Messzeitraum kontinuierlich, vorzugsweise linear, ab- oder zunimmt, wobei der jeweils andere Volumenstrom konstant gehalten wird, sodass zu Beginn des Messzeitraums ein unterstöchiometrisches oder überstöchiometrisches Gemisch und zum Ende des Messzeitraums ein überstöchiometrisches oder unterstöchiometrisches Gemisch vorliegt. Indem einer der beiden Volumenströme konstant gehalten wird, kann die Ermittlung des Luftbedarfs des Brenngases für eine stöchiometrische Verbrennung vereinfacht werden. Es ist besonders bevorzugt, wenn zu Beginn des Messzeitraums ein unterstöchiometrisches Gemisch vorliegt.

Dem Fachmann sind typische Verhältnisse von Luft und Brenngas, bei denen ein unter- oder überstöchiometrisches Gemisch vorliegt, bekannt. Dies gilt insbesondere für den Fall, in denen die Art des Brenngases, bspw. Erdgas, bekannt ist. Für ein unterstöchiometrisches Gemisch kann das Verhältnis zwischen Luftvolumenstrom und Brenngasvolumenstrom kleiner als 7:1, weiter vorzugsweise kleiner als 6:1 sein. Für ein überstöchiometrisches Gemisch kann das Verhältnis zwischen Luftvolumenstrom und Brenngasvolumenstrom größer als 12:1, vorzugweise größer als 14:1sein. Für H-Erdgas können die Verhältnisse zwischen Luftvolumenstrom und Brenngasvolumenstrom bspw. ca. 6:1 für ein unterstöchiometrisches Gemisch und ca. 15:1 für ein überstöchiometrisches Gemisch betragen.

Unabhängig davon, ob dem Fachmann die Art des Brenngases bekannt ist, kann er das erfindungsgemäße Verfahren zunächst mit typischen Verhältnissen, wie sie oben beispielhaft genannt sind, als angenommene Verhältnisse für über- bzw. unterstöchiometrische Gemische durchführen. Sollte für das Brenngas, dessen charakteristische Werten bestimmt werden sollen, während des Verfahrens keine stöchiometrische Verbrennung festgestellt werden können, kann das Verfahren solange mit anderen angenommenen Verhältnissen wiederholt werden, bis eine stöchiometrische Verbrennung festgestellt werden kann.

Vorzugsweise überwachte der Sensor die Flamme hinsichtlich der Temperatur und/oder der Ionisation. Sowohl Temperatur und Ionisation der Flamme sind bei stöchiometrischer Verbrennung maximal und somit größer als bei über- oder unterstöchiometrischer Verbrennung. Wird die Temperatur und/oder die Ionisation der Flamme im Messzeitraum überwacht, lässt sich über die Bestimmung des Zeitpunkts, an dem ein Maximalwert erreicht wird, das Verhältnis der Volumenströme von Luft und Brenngas und daraus der Luftbedarf des Brenngases ermitteln.

Weiter bevorzugt ist es, wenn der erfindungsgemäß ermittelte Luftbedarf mithilfe einer Kennlinie in einen Wobbeindex umgerechnet wird. Der Wobbeindex dient zur Beschreibung der Qualität von Brenngasen und ist für typische Erdgase in der Verteilung proportional zum Luftbedarf. Insbesondere wenn die grundsätzliche Art des Brenngases bekannt - also bspw. Erdgas - lässt sich über eine ggf. brenngasspezifische Kennlinie für die jeweilige Art von Brenngas aufgrund einer vorhandenen Proportionalität aus dem Luftbedarf ein Wobbeindex ermitteln. Auch wenn eine entsprechende Ermittlung des Wobbeindex nur eine Näherung an den tatsächlichen Wobbeindex darstellt, ist der so ermittelte Wert für viele Anwendungsbereiche ausreichend genau.

Liegen noch zusätzliche Informationen über das Brenngas vor, kann die Kennlinie in Abhängigkeit dieser Informationen angepasst werden, was im Ergebnis zu einer genaueren Bestimmung des Wobbeindex führen kann. Es ist daher bevorzugt, wenn die Kennlinie zur Umrechnung des Luftbedarfs in einen Wobbeindex in Abhängigkeit wenigstens eines weiteren Messwerts verändert wird. Bei den entsprechenden Messwerten kann es sich beispielsweise um den CO2-Gehalt des Brenngases und/oder den CH4-Gehalt des Brenngases, der der Bestimmung der relativen Dichte dienen kann, handeln. Die entsprechenden Messwerte können durch einen dafür geeigneten Sensor erfasst werden.

Das erfindungsgemäße Verfahren eignet sich insbesondere zur Bestimmung wenigstens eines Kennwerts von Erdgas. Es ist daher bevorzugt, wenn das Brenngas Erdgas ist. Weiter vorzugsweise handelt es sich bei dem Brenngas um H-Erdgas, das heißt Erdgas mit hohem Energiegehalt.

Die erfindungsgemäße Vorrichtung ist zur Durchführung des erfindungsgemäßen Verfahrens und ggf. dessen vorteilhafter Weiterbildungen ausgebildet. Es wird daher auch auf die vorstehenden Erläuterungen verwiesen.

Die erfindungsgemäße Vorrichtung zur Bestimmung wenigstens eines Kennwerts von Brenngasen umfasst zwei voneinander getrennte, veränderbare Messvolumen für Luft und Brenngas. Das eine Messvolumen ist also für Luft, das andere Messvolumen für Brenngas vorgesehen. Beide Messvolumen sind mit einem Brenner verbunden, sodass dort ein Gemisch von aus den jeweiligen Messvolumen zugeführter Luft und zugeführtem Brenngas entstehen und verbrannt werden kann. Zur Überwachung der dabei entstehenden Flamme ist ein Sensor im Flammenbereich des Brenners angeordnet. Dieser Sensor ist mit einer Steuervorrichtung verbunden.

Die Steuervorrichtung ist dazu ausgebildet, die beiden Messvolumen hinsichtlich ihres jeweiligen Volumens kontrolliert zu verändern. Durch entsprechende Volumenänderung der Messvolumen entstehen Volumenströme von Luft bzw. Brenngas, die jeweils den Brenner zugeführt werden. Die Steuervorrichtung steuert die Volumenänderung der beiden Messvolumen und damit die beiden Volumenströme in einem Messzeitraum derart, dass zu einem Zeitpunkt im Messzeitraum ein unterstöchiometrisches Gemisch von Luft und Brenngas, zu einem anderen Zeitpunkt im Messzeitraum ein überstöchiometrisches Gemisch vorliegt. Die Veränderung des oder der Volumenströme geschieht dabei kontinuierlich, sodass zumindest zu einem Zeitpunkt im Messzeitraum eine stöchiometrische Verbrennung im Brenner stattfindet.

Über den Sensor im Flammenbereich des Brenners lässt sich der Zeitpunkt der stöchiometrischen Verbrennung feststellen. Aus den jeweiligen Raten der Volumenänderung der beiden Messvolumen bzw. den daraus resultierenden Volumenströmen lässt sich für diesen Zeitpunkt das Verhältnis der Volumenströme von Luft und Brenngas ermitteln. Aus diesem Verhältnis ergibt sich dann der Luftbedarf.

Es ist bevorzugt, wenn wenigstens ein Messvolumen, vorzugsweise beide Messvolumen als Zylinder mit beweglichem Kolben ausgestaltet sind. Durch Bewegung des Kolbens lässt sich dann das jeweilige Messvolumen verändern. Für die entsprechende Bewegung des jeweiligen Kolbens ist vorzugsweise ein Schrittmotor vorgesehen. Schrittmotoren bieten den Vorteil, dass sie ohne aufwendige Sensorik hinsichtlich Geschwindigkeit und Position genau betrieben werden können. Sie sind weiterhin geeignet, die Kolben so anzutreiben, dass ein kontinuierlicher Volumenstrom entsteht. Die Vorschubgeschwindigkeit eines Kolbens kann dabei über den Durchmesser des Zylinders, in dem der Kolben angeordnet ist, in einen Volumenstrom umgerechnet werden. Um Ungenauigkeiten bei dieser Umrechnung so gering wie möglich zu halten, ist der Zylinder dabei vorzugsweise hochgenau hergestellt.

Vorzugsweise das Messvolumen für Luft 5- bis 15-mal, vorzugsweise 7- bis 12-mal, weiter vorzugsweise 10-mal größer als das Messvolumen für das Brenngas. Bei entsprechenden Volumenverhältnissen der Messvolumen ist gewährleistet, dass die erfindungsgemäße Bestimmung wenigstens eines Kennwerts von Brenngasen für typische Brenngase durchgeführt werden kann.

Bei dem Sensor im Flammenbereich des Brenners handelte sich bevorzugt um einen Sensor zur Messung der Temperatur und/oder der Ionisation der Flamme.

Weiter bevorzugt ist es, wenn im Messvolumen für das Brenngas ein Sensor für den CO2- und/oder CH4-Gehalt des Brenngases vorgesehen ist. Der Sensor kann mit dem Steuergerät verbunden sein. Ist das Steuergerät zur Ermittlung eines Wobbeindex anhand einer Kennlinie ausgebildet, kann dessen Genauigkeit durch Berücksichtigung der Messwerte des Sensors erhöht werden (siehe oben).

Die erfindungsgemäße Vorrichtung eignet sich insbesondere zur Bestimmung wenigstens eines Kennwerts von Erdgas. Bei dem Brenngas handelt es sich daher vorzugsweise um Erdgas, weiter vorzugsweise um H-Erdgas.

Die Erfindung wird nun anhand einer vorteilhaften Ausführungsform unter Bezugnahme auf die beigefügten Zeichnungen beispielhaft beschrieben. Es zeigen:
- Figur 1:: ein Ausführungsbeispiel einer erfindungsgemäße Vorrichtung zur Bestimmung wenigstens eines Kennwerts von Brenngasen;
- Figur 2:: ein beispielhafter Verlauf der Volumenströme und des Sensorsignals der Vorrichtung aus Figur 1; und
- Figur 3:: eine beispielhafte Kennlinie zur Ermittlung eines Wobbeindex aus dem Luftbedarf eines Brenngases.

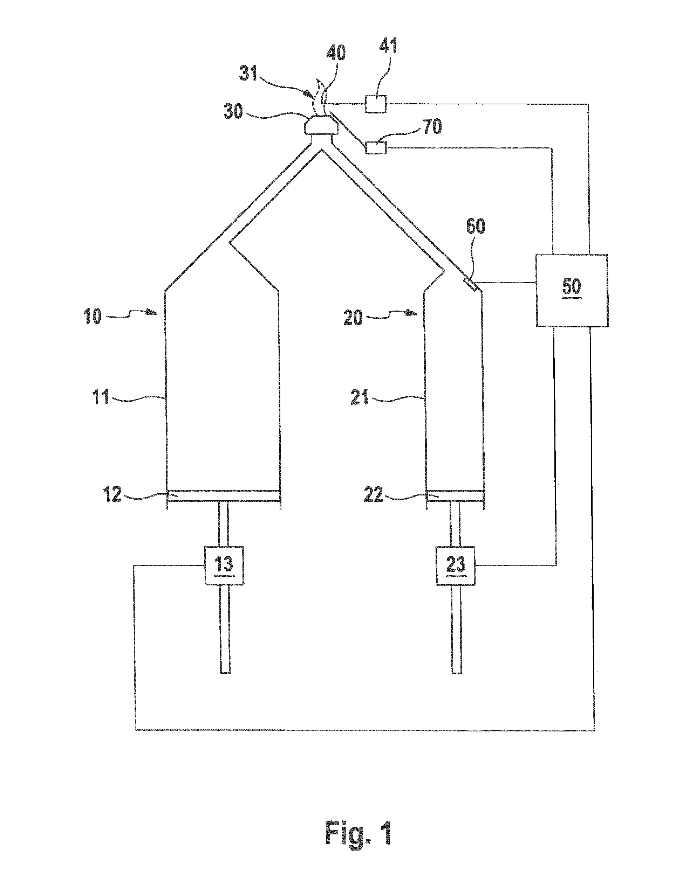

In Figur 1 ist ein erstes Ausführungsbeispiel einer erfindungsgemäßen Vorrichtung 1 zur Bestimmung wenigstens eines Kennwerts von Brenngases dargestellt. Die Vorrichtung 1 umfasst zwei Messvolumen 10, 20, von denen das eine Messvolumen 10 für die Aufnahme von Luft, das andere Messvolumen 20 für die Aufnahme dem Brenngas, von dem wenigstens ein Kennwert bestimmt werden soll, vorgesehen ist.

Die Messvolumen 10, 20 sind als Zylinder 11, 21 mit bewegbarem Kolben 12, 22 ausgestaltet. Die Kolben 12, 22 werden jeweils von einem Schrittmotor 13, 23 angetrieben. Die Schrittmotoren 13, 23 ermöglichen eine gute Steuerbarkeit der Bewegung der Kolben 12, 22, insbesondere hinsichtlich der Vorschubgeschwindigkeit, mit der die Kolben 12, 22 bewegt werden. Mit der Bewegung der Kolben 12, 22 wird das jeweilige Messvolumen 10, 20 verändert. Im Ausgangszustand ist das Messvolumen 10 für die Luft ca. 10-mal größer als das Messvolumen für das Brenngas.

Die Messvolumen 10, 20 sind beide mit einem Brenner 30 verbunden. Bei einer Veränderung der Messvolumen 10, 20, insbesondere einer Verkleinerung, wird ein Volumenstrom von Luft und/oder Brenngas aus dem jeweiligen Messvolumen 10, 20 zum Brenner 30 hin erzeugt, wobei die Größe des Volumenstroms abhängig von der Vorschubgeschwindigkeit des jeweiligen Kolbens 12, 22 ist. Am Brenner 30 werden die von den beiden Messvolumen 10, 20 stammenden Volumenströme vermischt, sodass ein Brenngas-Luft-Gemisch entsteht. Dieses Gemisch wird im Brenner 30 verbrannt.

Im Flammenbereich 31 des Brenners 30, also in dem Bereich, in dem sich bei laufender Verbrennung die Flamme des Brenners 30 befindet (in Figur 1 mithilfe einer gestrichelten Linie angedeutet), ist ein Sensor 40 angeordnet. Mit diesem Sensor 40 kann die Temperatur und/oder die Ionisation der Flamme ermittelt werden. Mit einer Sensorelektronik 41 werden die entsprechenden Messwerte aufbereitet.

Die Schrittmotoren 13, 23 sind ebenso wie der Sensor 40 mit der Steuerungsvorrichtung 50 verbunden. Die Steuerungsvorrichtung 50 ist dabei so ausgebildet, dass sie über die Schrittmotoren 13, 23 die Vorschubgeschwindigkeit der Kolben 12, 22 exakt steuern kann. Darüber hinaus kann die Steuerungsvorrichtung 50 die vom Sensor 40 erhaltenen Werte auswerten.

Die Funktionsweise der Vorrichtung 1 und insbesondere der Steuerungsvorrichtung 50 ergibt sich aus der nachfolgenden Erläuterung des erfindungsgemäßen Verfahrens zur Bestimmung wenigstens eines Kennwerts von Brenngasen, wie es mit der Vorrichtung 1 aus Figur 1 durchgeführt werden kann.

Im Ausgangszustand ist das Messvolumen 10 mit Luft gefüllt, während das Messvolumen 20 mit dem Brenngas, vom dem wenigstens ein Kennwert ermittelt werden sollen, gefüllt ist. Die Vorschubgeschwindigkeit der Kolben 12, 22 wird zu Beginn des Messzeitraums derart eingestellt, dass sich die daraus ergebenden Volumenströme aus Luft und Brenngas im Bereich des Brenners 30 ein Gemisch für eine unterstöchiometrische Verbrennung ergeben.

Im folgenden Messzeitraum bleibt die Vorschubgeschwindigkeit des Kolbens 12 und damit der Volumenstrom von Luft konstant, während die Vorschubgeschwindigkeit des Kolbens 11 verzögert wird. Durch diese Verzögerung nimmt der Volumenstrom von Brenngas über den Messzeitraum ab, wodurch sich auch das Verhältnis von Luft und Brenngas im Bereich des Brenners 30 verändert. Es ist vorgesehen, dass sich das Gemisch für eine unterstöchiometrische Verbrennung zu Beginn des Messzeitraums in ein Gemisch für eine überstöchiometrische Verbrennung zum Ende des Messzeitraums wandelt.

Bei dem dargestellten Ausführungsbeispiel wird zunächst von Verhältnissen zwischen Luftvolumenstrom und Brenngasvolumenstrom von 6:1 für ein unterstöchiometrisches Gemisch und 15:1 für ein überstöchiometrisches Gemisch ausgegangen. Für die meisten Brenngase sind diese angenommenen Verhältnisse zutreffend, sodass mit diesen Verhältnissen wird für die meisten Brenngase während des erfindungsgemäßen Verfahrens zu einem Zeitpunkt eine stöchiometrische Verbrennung erreicht wird. Sollte bei der erfindungsgemäßen Bestimmung wenigstens eines Kennwerts des zu überprüfenden Brenngasen mit diesen Werten dennoch keine stöchiometrische Verbrennung feststellen lassen, kann das Verfahren mit anderen angenommenen Verhältnissen solange wiederholt werden, bis eine stöchiometrische Verbrennung festgestellt werden kann.

Selbstverständlich ist es auch möglich, zunächst ein überstöchiometrisches Gemisch zu Beginn des Messzeitraumes einzustellen, welches dann in ein unterstöchiometrisches Gemisch gewandelt wird. Aus Gründen der Übersichtlichkeit werden im Folgenden jedoch nur das Beispiel eines überstöchiometrischen Gemisches zu Beginn und ein unterstöchiometrisches Gemisch zum Ende des Messzeitraums näher beschrieben.

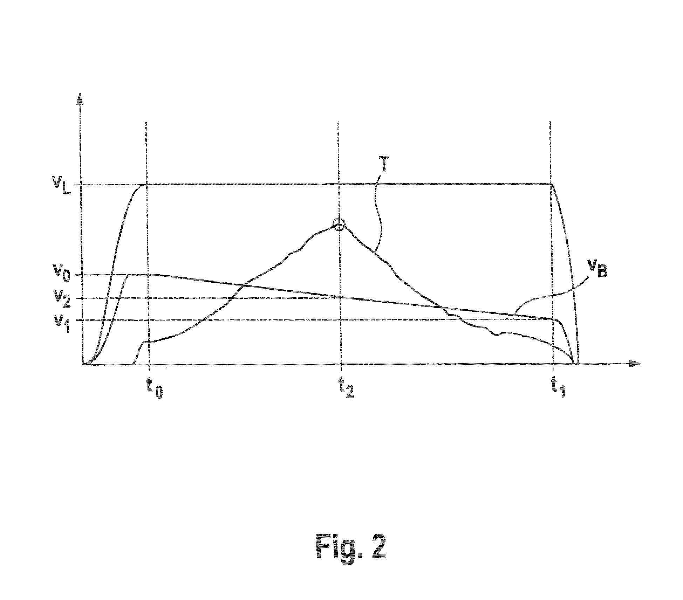

Der Flammenbereich 31 des Brenners 30 bzw. die Flamme wird durch den Sensor 40 während des Messzeitraums hinsichtlich der Temperatur und/oder Ionisation überwacht. In Figur 2 ist ein typischer Verlauf der Messergebnisse des Sensors 40 über den Messzeitraum dargestellt.

Wie in Figur 2 ersichtlich, wird die Vorschubgeschwindigkeit v_{L} des Kolbens 12 im mit Luft gefüllten Zylinder 11 über den Messzeitraum t₀ bis t₁ konstant gehalten. Die Vorschubgeschwindigkeit v_{B} des Kolbens 22 im mit Brenngas gefüllten Zylinder 21 wird von einer Geschwindigkeit v₀ zu Beginn des Messzeitraums t₀ linear bis auf eine Geschwindigkeit v₁ zum Ende des Messzeitraums t₁ verzögert. Die Geschwindigkeiten v₀ und v₁ sind dabei so gewählt, dass sich im Bereich des Brenners zu Beginn des Messzeitraums t₀ ein unterstöchiometrisches Gemisch aus Luft und Brenngas, zum Ende des Messzeitraums t₁ ein überstöchiometrisches Gemisch ergibt. Die in Figur 2 gezeigten Geschwindigkeitsverläufe außerhalb des Messzeitraums t₀ bis t₁ stellen die Beschleunigung und das Abbremsen der Kolben 12, 22 dar, um die gewünschten Geschwindigkeitsverhältnisse zu Beginn und am Ende des Messzeitraums t₀ bis t₁ zu gewährleisten.

Während des gesamten Messzeitraums t₀ bis t₁ wird das aus den, durch die Bewegung der Kolben 12, 22 erzeugten Volumenströmen entstehende Gemisch im Brenner 30 verbrannt. Der Temperaturverlauf T dieser Flamme wird über den Sensor 40 erfasst und ist in Figur 2 beispielhaft dargestellt. Wie in Figur 2 ersichtlich, weist die Kurve für die Temperatur T im Flammenbereich 31 des Brenners 30 ein Maximum zum Zeitpunkt t₂ auf. Aufgrund des erfindungsgemäßen Verfahrens, bei dem sowohl eine überstöchiometrische und eine unterstöchiometrische Verbrennung erfolgt, ergibt sich direkt, dass zum Zeitpunkt t₂ der maximalen Temperatur eine stöchiometrische Verbrennung erfolgt. Die Steuereinrichtung 50 kann dann aus den Vorschubgeschwindigkeiten der Kolben 12, 22 v_{L} und v₂ zum Zeitpunkt t₂ die jeweiligen Volumenströme und somit das Verhältnis von Luft und Brenngas im Gemisch, welches im Brenner 30 verbrannt wird, ermitteln. Aus diesem Verhältnis der Volumenströme lässt sich wiederum der Luftbedarf des Brenngases ermitteln.

Zur Umrechnung der Vorschubgeschwindigkeiten der Kolben 12, 22 in Volumenströme kann der Durchmesser der jeweiligen Zylinder 11, 21 herangezogen werden. Die Zylinder sind vorzugsweise hochgenau gefertigt, sodass Fehler bei der genannten Umrechnung minimiert werden.

Wenn anstelle der Temperatur T die Ionisation der Flamme durch den Sensor 40 erfasst wird, ergibt sich dennoch ein mit Figur 2 vergleichbares Bild. Die Ionisation der Flamme weist nämlich zum Zeitpunkt der stöchiometrischen Verbrennung - also zum Zeitpunkt t₂ - ebenfalls ein Maximum auf.

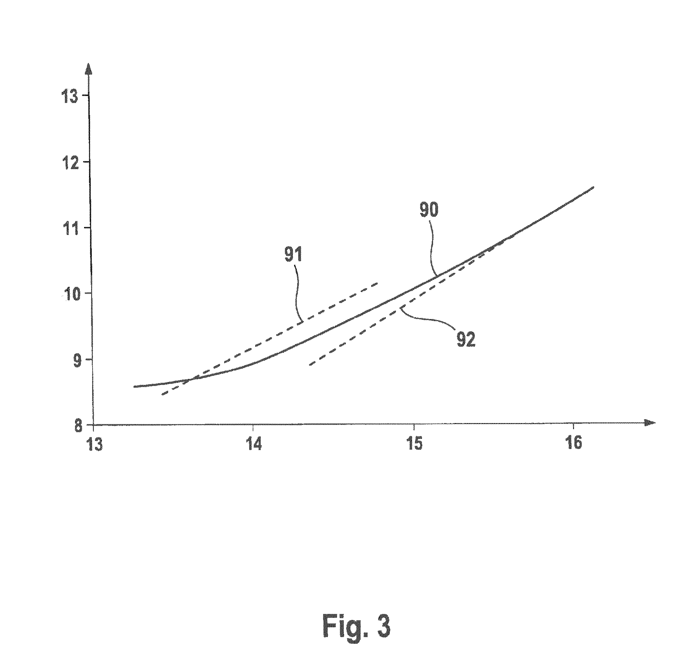

Ist der Luftbedarf bekannt, kann über eine Kennlinie der Wobbeindex des Brenngases ermittelt werden. Eine entsprechende Kennlinie 90 ist beispielhaft in Figur 3 gezeigt, wobei auf der x-Achse der Wobbeindex, auf der y-Achse der Luftbedarf eingetragen ist. Die Kennlinie 90 eignet sich, für beliebige Brenngase einen Näherungswert für den Wobbeindex zu bestimmen. Sind von dem Gas noch weitere Eigenschaften und Charakteristika bekannt, kann die Kennlinie 90 auch zu einer der Kennlinien 91, 92 verändert werden, die einen genaueren Wobbeindex bei ermitteltem Luftbedarf liefern.

Die Veränderung der Kennlinie 90 bspw. zu einer der Kennlinien 91, 92 kann auf Basis des CH4- und/oder CO2-Gehalts im Brenngas erfolgen. Dazu ist - wie in Figur 1 zu sehen - im Messvolumen 20, welches mit Brenngas gefüllt ist, ein Sensor 60 vorgesehen, mit dem der CH4- und/oder CO2-Gehalt des Brenngases ermittelt werden kann. Sind vom Brenngas entsprechende Werte bekannt und wird der Luftbedarf wie beschrieben ermittelt, lässt sich auch diesen Werten der Wobbeindex für das Brenngas mit höherer Genauigkeit ermitteln, als wenn dieser nur allein aufgrund des gemessenen Luftbedarfs ermittelt werden würde.

Die Vorrichtung in Figur 1 weit außerdem noch eine Zündvorrichtung 70 auf, die mit der Steuervorrichtung 50 verbunden ist. Mithilfe der Zündvorrichtung 70 kann die Steuervorrichtung 50 das aus dem Brenner30 ausströmende Brenngas-Luft-Gemisch automatisiert entzünden.

## Patentansprüche

1. Verfahren zur Bestimmung wenigstens eines Kennwerts von Brenngasen mit den Schritten:
a. Vermischen eines kontinuierlichen Volumenstroms von Luft und eines kontinuierlichen Volumenstroms eines Brenngases, wobei ein oder beide Volumenströme über einen Messzeitraum derart kontinuierlich verändert werden, dass zu einem ersten Zeitpunkt im Messzeitraum ein unterstöchiometrisches Gemisch und zu einem zweiten Zeitpunkt im Messzeitraum ein überstöchiometrisches Gemisch vorliegt;
b. Verbrennen des Gemisches bei gleichzeitiger Überwachung der Flamme durch einen Sensor (40); und
c. Ermitteln des Luftbedarfs des Brenngases aus dem Verhältnis der Volumenströme von Luft und Brenngas zum Zeitpunkt einer über den Sensor (40) festgestellten stöchiometrischen Verbrennung.

2. Verfahren nach Anspruch 1,
**dadurch gekennzeichnet, dass**
der Volumenstrom des Brenngases oder der Luft über den Messzeitraum kontinuierlich, vorzugsweise linear, ab- oder zunimmt, wobei der jeweils andere Volumenstrom konstant gehalten wird, sodass zu Beginn des Messzeitraums ein unterstöchiometrisches oder überstöchiometrisches Gemisch und zum Ende des Messzeitraums ein überstöchiometrisches oder unterstöchiometrisches Gemisch vorliegt.

3. Verfahren nach einem der vorhergehenden Ansprüche,
**dadurch gekennzeichnet, dass**
der Sensor (40) die Flamme hinsichtlich der Temperatur und/oder der Ionisation überwacht.

4. Verfahren nach einem der vorhergehenden Ansprüche,
**dadurch gekennzeichnet, dass**
der ermittelte Luftbedarf mithilfe einer Kennlinie (90, 91, 92) in einen Wobbeindex umgerechnet wird.

5. Verfahren nach Anspruch 4,
**dadurch gekennzeichnet, dass**
die Kennlinie (90, 91, 92) zur Umrechnung des Luftbedarfs in einen Wobbeindex in Abhängigkeit wenigstens eines weiteren Messwerts verändert wird.

6. Verfahren nach Anspruch 5,
**dadurch gekennzeichnet, dass**
ein weiterer Messwert der CO2-Gehalt des Brenngases und/oder ein weiterer Messwert der CH4-Gehalt des Brenngases ist, der zur Bestimmung der relativen Dichte dient, wobei der oder die weiteren Messwerte durch einen oder mehrere dafür geeignete Sensoren erfasst werden.

7. Verfahren nach einem der vorhergehenden Ansprüche,
**dadurch gekennzeichnet, dass**
das Brenngas Erdgas, vorzugsweise H-Erdgas, ist.

8. Vorrichtung zur Bestimmung wenigstens eines Kennwerts von Brenngasen umfassend zwei voneinander getrennte, veränderbare Messvolumen (10, 20) für Luft und Brenngas, die mit einem Brenner verbunden sind (30), ein im Flammenbereich des Brenners (31) angeordneter Sensor (40) und eine Steuervorrichtung (50), die mit dem Sensor (40) verbunden ist, wobei die Steuervorrichtung (50) zum Steuern der Volumenänderung der Messvolumen (10, 20) in einem Messzeitraum ausgebildet ist, sodass Luft und Brenngas jeweils als kontinuierlicher Volumenstrom den Brenner zugeführt und ein oder beide Volumenströme im Messzeitraum derart kontinuierlich verändert werden, dass zu einem Zeitpunkt im Messzeitraum ein unterstöchiometrisches Gemisch und zu einem anderen Zeitpunkt im Messzeitraum ein überstöchiometrisches Gemisch vorliegt und der Luftbedarf des Brenngases aus dem Verhältnis der Volumenströme von Luft und Brenngas zum Zeitpunkt einer über den Sensor (40) festgestellten stöchiometrischen Verbrennung ermittelt wird.

9. Vorrichtung nach Anspruch 8,
**dadurch gekennzeichnet, dass**
wenigstens ein Messvolumen (10, 20), vorzugsweise beide Messvolumen (10, 20), als Zylinder (11, 21) mit beweglichem Kolben (12, 22) ausgestaltet sind und vorzugsweise jeweils ein Schrittmotor (13, 23) für die Bewegung eines Kolbens (12, 22) vorgesehen ist.

10. Vorrichtung nach Anspruch 8 oder 9,
**dadurch gekennzeichnet, dass**
das Messvolumen für Luft 5- bis 15-mal, vorzugsweise 8- bis 12-mal, weiter vorzugsweise 10-mal größer als das Messvolumen für das Brenngas.

11. Vorrichtung nach einem der Ansprüche 8 bis 10,
**dadurch gekennzeichnet, dass**
der Sensor (40) zur Messung der Temperatur und/oder der Ionisation ausgebildet ist.

12. Vorrichtung nach einem der Ansprüche 8 bis 11,
**dadurch gekennzeichnet, dass**
im Messvolumen (20) für das Brenngas ein Sensor (60) für den C02- und/oder CH4-Gehalt des Brenngases vorgesehen ist.

13. Vorrichtung nach einem der Ansprüche 8 bis 12,
**dadurch gekennzeichnet, dass**
die Steuervorrichtung (50) zur Durchführung des Verfahrens nach einem der Ansprüche 2, 4 oder 5 ausgebildet ist.

14. Vorrichtung nach einem der Ansprüche 8 bis 13,
**dadurch gekennzeichnet, dass**
das Brenngas Erdgas, vorzugsweise H-Erdgas, ist.
